# EUROPÄISCHE PATENTANMELDUNG

(11) **EP 1 293 175 A2**
(43) Veröffentlichungstag der Anmeldung: **19.03.2003**
(21) Anmeldenummer: 02009366.2
(22) Anmeldetag: 03.05.2002
(51) Int. Cl.: A61C 19/00

(54) **Lichthärtgerät sowie Lichtquelle**

(30) Priorität: 06.06.2001 DE 10127416
(71) Anmelder: Firma Ivoclar Vivadent AG, 9494 Schaan (LI)
(72) Erfinder: Planck, Wolfgang, 6830 Rankweil (AT); Rohner, Gottfried, 9450 Altstätten (CH); Stahl, Thomas, 6900 Bregenz (AT)
(74) Vertreter: Baronetzky, Klaus, Dipl.-Ing.

(57) **Zusammenfassung**

Ein Lichthärtgerät weist eine Lichtquelle, die eine Mehrzahl von LEDs aufweist, die von einer gemeinsamen Stromquelle gespeist sind, und eine Kühlvorrichtung auf, die hinter der Lichtquelle angeordnet ist und für deren Kühlung bestimmt ist. Die LED-Anordnung ist auf der Stirnfläche eines Kühlkörpers gelagert, der Anschlüsse wie eine Halogen-Glühlampe aufweist.

## Beschreibung

Die Erfindung betrifft ein Lichthärtgerät, gemäß dem Oberbegriff von Anspruch 1 sowie eine Lichtquelle gemäß dem Oberbegriff von Anspruch 17.

Ein deratiges Lichthärtgerät ist beispielsweise aus der US-PS 5 420 768 bekannt. Bei dieser Lösung ist eine Mehrzahl von Leuchtdioden auf einem gemeinsamen Basiskörper angebracht. Das von den Leuchtdioden emittierte Licht wird einem Lichtleiter zugeleitet und kann beispielsweise zur Lichthärtung von hierfür geeigneten polymerisierbaren Massen eingesetzt werden, beispielsweise im Dentalbereich.

Ferner ist es vorgeschlagen wurden, eine Kühlung des Basiskörpers für die Leuchtdioden in geeigneter Weise vorzunehmen.

Es ist auch bereits vorgeschlagen wurden, als Basiskörper einen Kühlkörper zu verwenden, wozu beispielsweise auf die Deutsche Patentanmeldung 101 04 579 zu verweisen ist. Gemäß diesem Vorschlag ist ein Kühlkörper vorgesehen, der auf seiner Basisfläche zahlreiche LEDs lagert. Der Kühlkörper ist von Rippenkörpern umschlossen, und das austretenden Licht wird einem Lichtleiter zugeleitet, teilweise nachdem es von dem vorderen, als gegen ausgebildeten Rippenkörper, reflektiert worden ist.

Diese Lösung bietet zwar grundsätzlich einer recht guten Kühlung der LED-Anordnung.

Wenn allerdings zur Erzielung einer maximalen Lichtausbeute die Basisfläche des Kühlkörpers mit eng aneinandergereihten LED-Chips bestückt ist, wird der Kühlkörper bei intensiver Außenkühlung zu heiß, so daß gemäß der vorgeschlagenen Lösung ein bestimmter Abstand zwischen den LED-Chips eingehalten wird. Die Chips umgebende Freifläche dient zugleich als Reflektionsfläche, um die von dem innen verspiegelten Gegenkonus (der dem vorderen Rippenkörper entspricht) zurückgeworfene Strahlung erneut zu reflektieren und dem Lichtleiter zuzuleiten.

Diese Lösung führt überraschend dazu, das gerade der vordere Rippenkörper recht heiß wird, so daß bereits eine Wasserkühlung vorgeschlagen wurde. Mit einer Wasserkühlung läßt sich zwar der Kühleffekt deutlich steigern, jedoch erhöht sie das Gewicht eines Lichthärtgeräts, das als Handgerät dann unhandlich schwer wird.

Daher liegt der Erfindung die Aufgabe zugrunde, ein Lichthärtgerät gemäß dem Oberbegriff von Anspruch 1 sowie eine Lichtquelle gemäß dem Oberbegriff von Anspruch 17 zu schaffen, die hinsichtlich des Lichtwirkungsgrads, also das Verhältnis zwischen abgegebener Lichtleistung und abgegebener Abwärme, verbessert ist.

Diese Aufgabe wird erfindungsgemäß durch Anspruch 1 gelöst. Vorteilhafte Weiterbildungen ergeben sich aus den Unteransprüchen.

Die erfindungsgemäße Lösung bietet überraschend die Möglichkeit, trotz Reduktion der Basisfläche, die für die Aufnahme der LED-Chips zur Verfügung steht, den Wirkungsgrad zu verbessern. Durch die Anordnung an der Stirnfläche können die LED-Chips dem Lichtleiter direkt benachbart angeordnet sein und Licht dort einleiten. Der Kühlkörper weitet sich bevorzugt von der Stirnfläche ausgehend nach hinten auf, so daß die für die Wärmeübertragung zur Verfügung stehende Masse und Fläche - betrachtet gegenüber dem Querschnitt der Stirnfläche drastisch vergrößert ist. Erfindungsgemäß besonders günstig ist es, daß durch die Anordnung an der Stirnfläche, also dem Lichtleiter unmittelbar benachbart, Reflexionsflächen nicht vorgesehen sein müssen. Bei den bislang bekannten Lösungen mit auf dem Kühlkörper aufgebrachten LED-Chips wird das Licht zum großen Teil Reflexionsflächen zugeleitet, dort reflektiert und nach ggf. mehrfacher weiterer Reflexionen schließlich dem Lichtleiter zugeleitet. Untersuchungen im Zusammenhang mit der Erfindung haben gezeigt, daß durch die Reflexionen nicht nur der Lichtwirkungsgrad reduziert wird, sondern daß dort auch eine erhebliche Verlustwärme entsteht, die abgeführt werden muß.

Erfindungsgemäß ist es demgegenüber vorgesehen, reflexionsfrei unter direkter Lichtbeaufschlagung zu arbeiten. Die von den LED-Chips abgegebene Wärmestrahlung wird zugleich auch über den Lichtleiter emittiert, so daß die erfindungsgemäße Lösung auch für Polymerisationsmassen geeignet sind, die auch auf Wärmestrahlung ansprechen.

Erfindungsgemäß ist es vorgesehen, daß die Mehrfach-LED-Anordnung als eine Art "Heißer Fleck" in dem Lichthärtgerät konzentriert Licht sowie die hierbei anfallende Verlustwärme abgibt. Durch die großflächige Wärmeverteilung über den konusförmigen Kühlkörper läßt sich gewährleisten, daß das Lichthärtgerät insgesamt nicht zu stark erwärmt wird.

Hinzu kommt, daß durch die erfindungsgemäß besonders hohe Lichtausbeute auch eine rasche Durchhärtung möglich ist. Die Härtungszeit kann bspw. gegenüber einer handelsüblichen Halogen-Glühlampen-Härtung um beispielsweise die Hälfte reduziert werden. Durch die verkürzte Härtezeit spielen andererseits auch die Wärmekapazität des Kühlkörpers eine Rolle, die zur Absenkung des Temperaturniveaus führt. Dies bedeutet praktisch, daß während der Härtezeit die Temperaturerhöhung außen am Lichthärtgerät unmerklich ist, und daß die Kühlvorrichtung auch über den Abschluß des Härtevorgangs hinaus die Kühlung vornimmt, so daß das Temperatur über die Zeit betrachtet eingeebnet wird.

Erfindungsgemäß besonders günstig ist es jedoch, daß die LED-Anordnung in Kombination mit dem Kühlkörper auch als Ersatz für die Reflektor-Halogen-Glühlampe eines handelsüblichen Lichthärtgeräts eingesetzt werden kann. Mit dieser Lösung läßt sich die Lichtleistung wesentlich erhöhen und die Lebensdauer und damit auch die Zuverlässigkeit des Lichthärtgeräts wird automatisch gesteigert. Die bislang problematische Härtung beim Durchbrennen einer Glühlampe, die dazu führt, daß der Patient mit halb gehärteter Füllung oder dergl. warten muß, läßt sich vermeiden. Bislang wurden häufig zur Vermeidung der ungünstigen Situation, daß eine Weiterhärtung einer halbgehärteten Füllung oder eines anderen Dentalrestaurts nicht möglich ist, sicherheitshalber zwei Lichthärtgeräte in der Zahnartztpraxis vorgehalten, so daß die Fertighärtung mit dem Ersatzgerät vorgenommen werden konnte. Erfindungsgemäß läßt sich dies vermeiden, nachdem die LED-Anordnung eine drastisch erhöhte Lebensdauer hat und ein vollständiger Ausfall praktisch ausgeschlossen ist. Dies liegt auch in der Mehrfach-Anordnung von LED-Chips begründet, denn wenn tatsächlich einmal ein Chip ausfällt, wird die Lichtleistung um beispielsweise 5% reduziert, was ohne weiteres durch eine erhöhte Härtezeit ausgelichen werden kann.

Es versteht sich, daß die Anzahl der LED-Chips in weiten Bereichen an die Erfordernisse anpassbar ist. Bei runden Fassungen für den Lichtleitstab kann beispielsweise eine Anordnung von 5 mal 5 LED-Chips gewählt werden, so daß insgesamt 25 Chips vorliegen, oder es können hier die in den Ecken vorgesehen Chips weggelassen werden, so daß 21 Chips vorliegen. Bevorzugt sind die Chips in dichtester Packung angeordnet und sind dem Eingang des Lichtleitstabs unmittelbar benachbart.

Erfindungsgemäß besonders günstig ist es, daß bei handelsüblichen Lichtleitgeräten keine weiteren Modifikationen am Lichthärtgerät selbst vorgenommen werden müssen, um die erfindungsgemäße Lichtquelle anstelle der Reflektor-Halogen-Glühlampe einzusetzen. Hierzu weist der Kühlkörper bevorzugt an seinem rückwärtigen Ende zwei elektrische Kontaktstifte auf, die den Kontaktstiften einer Halogen-Glühlampe entsprechen. Die Kühlung erfolgt in der einer Halogen-Glühlampe entsprechenden Weise durch Kontakt mit den Rippenkörpern, die die Halogen-Glühlampe ansonsten umgeben. Bevorzugt werden die beiden Rippenkörper so fest miteinander verbunden, daß eine sichere Anlage gewährleistet ist, so daß die Wärmeableitung gewährleistet ist.

Auch wenn grundsätzlich eine Wasserkühlung der Rippenkörper in Betracht kommt, so daß die Rippen sich innenliegend erstrecken, ist es bevorzugt, eine Luftkühlung mit einem Gebläse vorzusehen, was zu Gewichtsvorteilen führt.

Besonders günstig ist es auch, daß das Temperaturmanagement eines handelsüblichen Lichthärtgeräts ohne Modifikationen weiterverwendet werden kann. Üblicherweise wird das Gebläse eingeschaltet, sobald die Temperatur einen gewissen Schwellwert überschreitet. Aufgrund des verbesserten Wirkungsgrades liegt eine geringere Wärmeentwicklung vor, so daß das Gebläse entsprechend verzögert einschaltet.

Die erfindungsgemäße Lösung ist sowohl für Netzspannungs-Versorgung als auch für Versorgung mit einen Akkumulatorsatz geeignet. Durch den recht hohen Wirkungsgrad wird die Lebensdauer der Akkumulatoren verlängert.

Erfindungsgemäß ist es besonders günstig, wenn die LED-Anordnung aus Chips von mindestens zwei unterschiedlichen Typen aufgebaut ist, deren Emmisionsmaximum bei unterschiedlichen Wellenlängen liegt. Hiermit kann den Besonderheiten auch von Zweikatalysatorsystemen Rechnung getragen werden.

In vorteilhafter Ausgestaltung ist es ferner vorgesehen, den Kühlkörper hohl auszubilden und im Inneren eine Steuerschaltung für die LED-Chips aufzunehmen. Bei einer entsprechenden Modifikation der Software oder ggf. auch der Hardware des Handgeräts als Lichthärtgerät ist auch möglich, die beiden LED-Chiparten sequenziell einzuschalten, auch wenn lediglich zwei Stromversorgungskontakte vorgesehen sind. Hierzu kann bspw. eine Impulssteuerung vorgesehen sein, die über die in dem Kühlkörper integrierte Steuerschaltung zwischen den Chipsätzen umschaltet.

Es versteht sich, daß beliebige Modifikationen der erfindungsgemäßen Lösung möglich sind, ohne den Bereich der Erfindung zu verlassen. So kann der Kühlkörper bei Bedarf auch auch als Basiskühlkörper ausgebildet sein, der über entsprechende Adapter in gute Wärmeleitverbindung mit den Rippenkörpern von Lichthärtgeräten verschiedener Hersteller bringbar ist. Auch ist es möglich, den Wärme-Übergangswiderstand zwischen Kühlkörper und Rippenkörper durch den Einsatz von an sich bekannter Wärmeleitpaste zu verbessern.

Weitere Vorteile, Einzelheiten und Merkmale ergeben sich aus der nachfolgenden Beschreibung eines Ausführungsbeispiels der Erfindung anhand der Zeichnung.

Es zeigen:
- Fig. 1: eine schematische Seitenansicht eines erfindungsgemäßen Lichthärtgeräts, wobei eine Gehäusehälfte weggelassen ist und der Gegenrippenkörper abgenommen ist; und
- Fig. 2: eine vergrößerte Darstellung einer erfindungsgemäßen Lichtquelle für den Einsatz in der Ausführungsform des in Fig. 1 dargestellten Lichthärtgeräts.

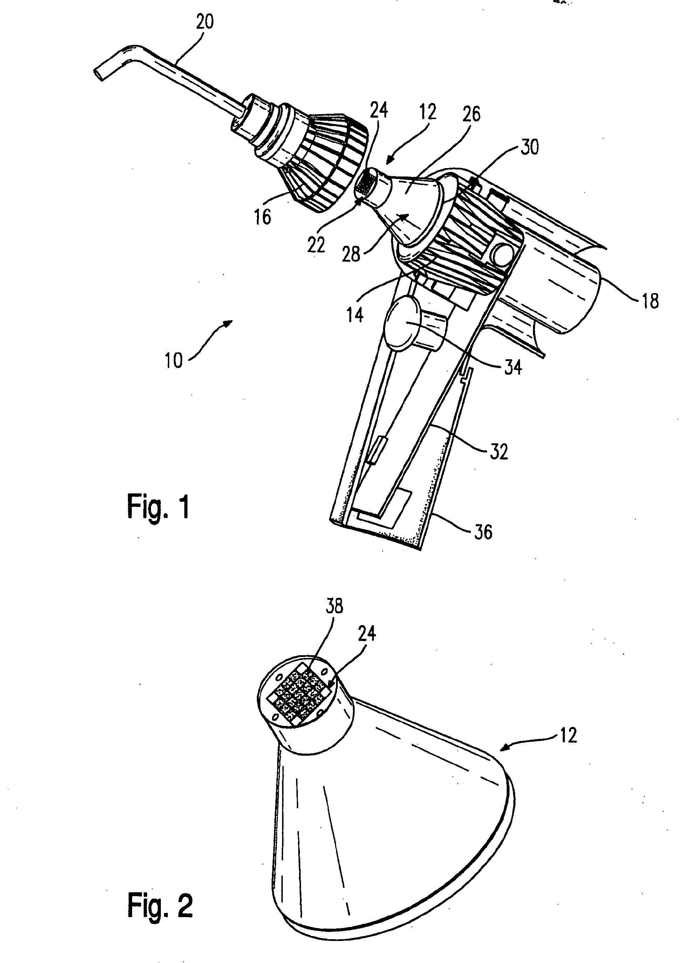

Das in Fig. 1 dargestellte Lichthärtgerät 10 weist eine Lichtquelle 12 auf, die zwischen einem Rippenkörper 14 und einem Gegenrippenkörper 16 eingeschlossen ist. In der Darstellung gemäß Fig. 1 ist der Gegenrippenkörper abgenommen dargestellt.

Beide Rippenkörper weisen ineinander übergehende Längsrippen auf, so daß sie durch eine Luftströmung von vorne nach hinten gekühlt werden können. Hierzu ist als Kühlvorrichtung 18 ein Gebläse vorgesehen, das hinter dem Rippenkörper 14 angeordnet ist.

Der Gegenrippenkörper 16 weist einen Anschluß für ein Lichtleitelement 20 auf, das sich in an sich bekannter Weise Lichtleitstab nach vorne erstreckt. Unmittelbar dem Eingang des Lichtleitelements 20 benachbart, ist eine LED-Anordnung 22 auf einer Stirnfläche 24 eines Kühlkörpers 26 aufgebracht. Die Aufbringung erfolgt in an sich bekannter Weise in guter Wärmeleitverbindung, beispielsweise mit Silberlot oder dergl. Die Stirnfläche 24 weist einen Querschnitt auf, der lediglich etwas größer als der Querschnitt des Lichtleitstabs ist und ebenfalls wesentlich kleiner als der maximale Querschnitt des Kühlkörpers 26, bspw. um den Faktor 5 bis 10.

Der Kühlkörper 26 ist in dem dargestellten Ausführungsbeispiel im wesentlichen konisch ausgebildet und weist eine Mantelfläche 28 auf, deren Gestalt an die Innenfläche des Gegenrippenkörpers 16 angepaßt ist.

Von einem Bereich maximalen Durchmessers 30 aus erstreckt sich der Kühlkörper 26 weiter nach hinten in den Rippenkörper 14 hinein, auch wenn dies aus Fig. 1 nicht ersichtlich ist. Dort läuft er konisch oder ballig zu, und weist eine Formgebung auf, die der Außenform einer handelsüblichen Halogen-Glühlampe im wesentlichen entspricht. Diese Form ist ausgelegt, daß sie zugleich einen minimalen Wärme-Übergangswiderstand gegenüber dem Rippenkörper 14 bietet.

Der Kühlkörper 26 endet in zwei nicht dargestellten Steckstiften, die den Steckstiften einer Reflektor-Halogen-Glühlampe entsprechen. Mit dieser Lösung und zentral durch den Kühlkörper 26 isoliert hindurchgeführten Anschlußdrähten ist es möglich, die LED-Anordnung 22 mit der Speisespannung, die für die Halogen-Glühlampe bestimmt ist, zu versorgen.

Das Lichthärtgerät 10 im übrigen weist an sich bekannte Bauteile auf, wie beispielsweise eine Elektronikplatine 32, die die Fassung für den elektrischen Anschluß der Lichtquelle 12 aufnimmt und zugleich eine Tastschalter 34 lagert. Das Lichthärtgerät 10 weist ferner ein an sich bekanntes pistolenförmiges Gehäuse 36 auf.

Aus Fig. 2 ist der Aufbau einer Lichtquelle 12 in vergrößerter Darstellung ersichtlich. Die Lichtquelle weist auf ihrer Stirnfläche 24 21 LED-Chips 38 auf, die dicht aneinander angrenzend gelagert sind. Der lichtemittierende Querschnitt ist der Kreisform durch Weglassen der Eck-Chips zumindest angenähert, so daß eine reflexionsfreie Einleitung des emittierten Lichts in das Lichtleitelement 20 möglich ist.

Auch wenn hier eine Anzahl von 21 Chips 38 dargestellt ist, sind bevorzugt 32 Chips in der Anordnung 6 mal 6, jedoch ohne die Eck-Chips vorgesehen. Hierdurch läßt sich eine besonders hohe Lichtleistung abgeben, und die elektrische Belastbarkeit eines üblichen Lichthärtgeräts mit einer 50 Watt-Halogenglühlampe läßt sich besser ausnutzen. Bei einer Durchlaßspannung von 3 Volt pro Chip können dann Gruppen von je 4 Chips in Reihe geschaltet werden, so daß 8 parallel geschaltete Chipgruppen vorliegen.

Diese können bei Bedarf auch mit unterschiedlichen LED-Chips bestückt sein, wobei auch über eine in den Kühlkörper integrierte Steuerschaltung eine selektive Anwahl der Chipgruppen möglich ist.

## Patentansprüche

1. Lichthärtgerät, mit einer Lichtquelle, die eine Mehrzahl von LEDs aufweist, die von einer elektrischen Energiequelle gespeist sind, mit einer Kühlvorrichtung, die hinter der Lichtquelle angeordnet ist und für deren Kühlung bestimmt ist, **dadurch gekennzeichnet, daß** die LED-Anordnung (22) auf der Stirnfläche (24) eines Kühlkörpers (26) gelagert ist, der Anschlüsse wie eine Halogen-Glühlampe aufweist.

2. Lichthärtgerät nach Anspruch 1, **dadurch gekennzeichnet, daß** der Kühlkörper (26) im wesentlichen konusförmig ist und die LED-Anordnung (22) an der Konusspitze angeordnet ist.

3. Lichthärtgerät nach einem der vorhergehenden Ansprüche, **dadurch gekennzeichnet, daß** der Kühlkörper (26) von Kühlmittel umströmt ist.

4. Lichthärtgerät nach einem der vorhergehenden Ansprüche, **dadurch gekennzeichnet, daß** das Licht der Lichtquelle (12) reflexionsfrei zu einem Lichtleitelement (20) gelangt.

5. Lichthärtgerät nach einem der vorhergehenden Ansprüche, **dadurch gekennzeichnet, daß** der Kühlkörper (26) in Wärmeleitver-bindung mit einem Rippenkörper (14, 16) angeordnet ist, der auf seiner Außenseite Kühlrippen aufweist.

6. Lichthärtgerät nach einem der vorhergehenden Ansprüche, **dadurch gekennzeichnet, daß** der Kühlkörper (26) an seiner von der LED-Anordnung (22) abgewandten Seite eine Form aufweist, die im wesentlichen der Außenform einer Reflektor-Halogen-Glühlampe entspricht.

7. Lichthärtgerät nach einem der vorhergehenden Ansprüche, **dadurch gekennzeichnet, daß** der Kühlkörper (26) an seiner von der LED-Anordnung (22) abgewandten Seite an einem Rippenkörper (14) in Wärmeleitverbindung anliegt.

8. Lichthärtgerät nach einem der vorhergehenden Ansprüche, **dadurch gekennzeichnet, daß** der Kühlkörper (26) aus Metall besteht und insbesondere innen hohl ist.

9. Lichthärtgerät nach einem der vorhergehenden Ansprüche, **dadurch gekennzeichnet, daß** der Kühlkörper (26) von Anschlußleitungen durchtreten ist, die an der von der Lichtquelle gegenüberliegenden Seite mit zwei Steckstiften verbunden sind, die in eine Halogenglühlampen-Fassung passen.

10. Lichthärtgerät nach einem der vorhergehenden Ansprüche, **dadurch gekennzeichnet, daß** die Kühlvorrichtung (18) als Gebläse ausgebildet ist.

11. Lichthärtgerät nach einem der vorhergehenden Ansprüche, **dadurch gekennzeichnet, daß** die LED-Anordnung (22) samt Kühlkörper (26) für eine Speisespannung zwischen 10 und 14 Volt ausgelegt ist und für den Austausch der Reflektor-Halogenglühlampe bestimmt ist.

12. Lichthärtgerät nach einem der vorhergehenden Ansprüche, **dadurch gekennzeichnet, daß** die LED-Anordnung (22) im Spektralbereich zwischen 350 und 600 Nanometer emittiert und daß insbesondere je eine Mehrzahl von LEDs vorgesehen sind, die Licht mit einem Emissionsmaximum bei einer kürzeren Wellenlänge und Licht bei einem weiteren Emissionsmaximum bei einer längeren Wellenlänge abstrahlen.

13. Lichthärtgerät nach einem der vorhergehenden Ansprüche, **dadurch gekennzeichnet, daß** die Lichtquelle (12) eine Leistung von mehr als 400 Milliwatt pro qcm, vorzugsweise 800 Milliwatt pro qcm und insbesondere etwa 1000 Milliwatt pro qcm abgibt.

14. Lichthärtgerät nach einem der vorhergehenden Ansprüche, **dadurch gekennzeichnet, daß** der Kühlkörper (26) einen Impuls/Pausen-Geber aufnimmt, mit welchem die LED-Anordnung intermittierend ansteuerbar ist.

15. Lichthärtgerät nach einem der vorhergehenden Ansprüche, **dadurch gekennzeichnet, daß** der Kühlkörper (26) samt LED-Anordnung (22) in einer Kombination aus einem Rippenkörper (14)und einem Gegen-Rippenkörper (16) einspannbar ist, die aneinender befestigbar, insbesondere verschraubbar sind.

16. Lichthärtgerät nach einem der vorhergehenden Ansprüche, **dadurch gekennzeichnet, daß** eine Mehrzahl von LEDs auf der Stirnfläche (24) in Matrixform angeordnet ist, wobei die LEDs parallel zueinander und in Reihe zueinander zur Festlegung einer Betriebsspannung zwischen 10 und 14 Volt verschaltet sind und die Stirnfläche im wesentlichen ausfüllen.

17. Lichtquelle mit einer Mehrzahl von LEDs, **dadurch gekennzeichnet, daß** sie auf einem Kühlkörper (26) angebracht ist, der Steckanschlüsse und eine Außenform aufweist, die denen einer handelsüblichen Reflektor-Halogen-Glühlampe mit einer Nennspannung von 12 Volt entsprechen.

18. Lichtquelle nach Anspruch 17, **gekennzeichnet durch** die kennzeichnenden Merkmale eines der Ansprüche 2 bis 16.
